# EUROPEAN PATENT SPECIFICATION

(11) **EP 1 070 726 B1**
(45) Date of publication and mention of the grant of the patent: **20.02.2008**
(21) Application number: 99932431.2
(22) Date of filing: 11.03.1999
(51) Int. Cl.: A23L 1/236, A23G 4/00, A23L 1/326, A23P 1/08, A23G 3/00, A23L 1/22, C07C 229/00, C07C 229/36

(54) **ASPARTYL DIPEPTIDE ESTER DERIVATIVES AND SWEETENERS**
ASPARTYLDIPEPTIDESTER-DERIVATE UND SÜSSUNGSMITTEL
DERIVES D' ESTERS D'ASPARTYLDIPEPTIDE ET EDULCORANTS

(30) Priority: 09.04.1998 JP 9770198; 17.02.1999 JP 3819099
(43) Date of publication of application: 24.01.2001
(73) Proprietor: Ajinomoto Co., Inc., Tokyo 104-8315 (JP)
(72) Inventor: Amino, Yusuke, Kawasaki-shi, Kanagawa-ken 210-0801 (JP); Yuzawa, Kazuko, Kawasaki-shi, Kanagawa-ken 210-0801 (JP); Takemoto, Tadashi, Kawasaki-shi, Kanagawa-ken 210-0801 (JP); Nakamura, Ryoichiro, Kawasaki-shi, Kanagawa-ken 210-0801 (JP)
(74) Representative: Nash, David Allan
(86) International application number: PCT/JP1999/001210
(87) International publication number: WO 1999/052937

(56) References cited:
- JP-A- 8 503 206

## Description

### TECHNICAL FIELD

The present invention relates to novel aspartyl dipeptide ester derivatives, and a sweetener and products such as foods having a sweetness, which contain the same as an active ingredient.

### BACKGROUND ART

In recent years, as eating habits have been improved to a high level, fatness caused by excessive intake of sugar and diseases accompanied by fatness, have been at issue. Accordingly, the development of a low-calory sweetener that replaces sugar has been in demand. As a sweetener that has been widely used at present, there is aspartame which is excellent in a safety and taste properties. However, this is somewhat problematic in the stability. In WO 94/11391, it is stated that derivatives in which an alkyl group is introduced in an amino group of aspartic acid constituting aspartame markedly improves sweetening potency and the stability is slightly improved. It is reported that the best compound described in this document is N-[N-(3,3-dimethylbutyl)-L-α -aspartyl] -L-phenylaianine 1-methyl ester having a 3,3-dimethylbutyl group as an alkyl group and the sweetening potency thereof is 10,000 times. Aspartame derivatives having introduced therein 20 types of substituents other than the 3,3-dimethylbutyl group are indicated therein, and the sweetening potency thereof is reported to be less than 2,500 times. Derivatives having a 3-(substituted phenyl)propyl group as an alkyl group are also shown. However, it is reported that the sweetening potency of N-[N-(3-phenylpropyl)-L-α-aspartyl]-L-phenylalanine 1-methyl ester is 1,500 times and that of N-[N-[3-(3-methoxy-4-hydroxyphenyl)propyl]-L-α-aspartyl] -L-phenylalanine 1-methyl ester is 2,500 times. Thus, these are far less than that (10,000 times) of N-[N-(3,3-dimethylbutyl)-L-α-aspartyl]-L-phenylalanine 1-methyl ester.

### PROBLEMS INVENTION IS TO SOLVE OBJECT THEREOF

It is an object of the invention to provide novel aspartyl dipeptide ester derivatives which are excellent in the safety and which have sweetening potency equal to or higher than that of the N-[N-(3,3-dimethylbutyl)-L-α-aspartyl]-L-phenylalanine 1-mehtyl ester, and a low-calory sweetener containing the same as an active ingredient.

### DISCLOSURE OF INVENTION

In order to solve the problems, the present inventors have synthesized several aspartame derivatives in which various 3-(substituted phenyl)propyl groups are introduced in an amino group of aspartic acid constituting the aspartame derivatives by use of cinnamaldehyde having various substituents on 3-phenylpropianaldehyde having various substituents that can easily derived therefrom an precursor aldehydes, and have examined the sweetening potency of them. They have consequently found that- with respect to the sweetening potency, the novel compounds that they have found are by far higher than not only N-[N-(3-phenylpropyl)-L-α-aspartyl]-L-phenylalanine 1-methyl ester which is reported to have the sweetening potency of 1,500 times in WO 94/11391 but also N-[N-(3,3-dimethylbutyl)-L-α-aspartyl]-L-phenylalanine 1-methyl ester which is reported therein to have the sweetening potency of 10,000 times, and that especially the compounds represented by the following formula (1) are excellent as a sweetener. These findings have led to the completion of the invention.

The present invention (Claim 1) is directed to novel aspartyl dipeptide ester derivatives (including those in the form of a salt) represented by the general formula (1): 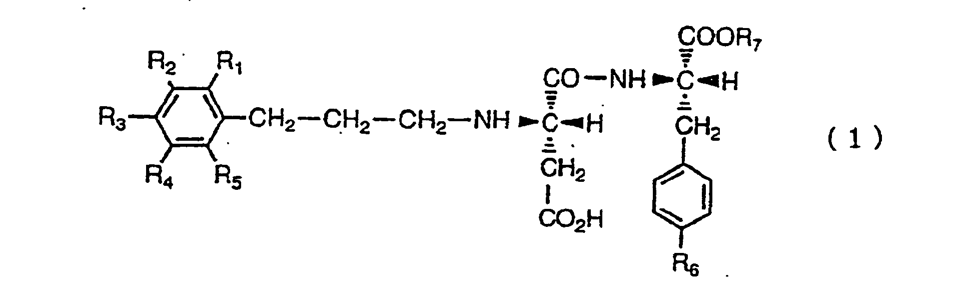 wherein
R₁, R₂, R₄ and R₅, independently from each other, represent a substituent selected from a hydrogen atom (H) , a hydroxyl group (OH) , an alkoxy group (OR; methoxy group, ethoxy group, propoxy groups, or the like) having from 1 to 3 carbon atoms, an alkyl group (R; methyl group, ethyl group, propyl groups, or the like) having from 1 to 3 carbon atoms and a hydroxyalkyloxy group (for examples, O (CH₂)₂OH or OCH₂CH (OH) CH₃) having 2 or 3 carbon atoms, or R₁ and R₂ together form a methylenedioxy group (OCH₂O) wherein R₁ and R₅ independently from each other, represent any substituent as mentioned above designated for the R₄ and R₅ radicals respectively;
R₃ is methoxy; and R₆ represents a hydrogen atom or a hydroxyl group, and R₇ represents a substituent selected from a methyl group (CH₃), an ethyl group (CH₂CH₃), an isopropyl group (CH(CH₃)₂, an n-propyl group (CH₂CH₂CH₃) and a t-butyl group (C (CH₃) ₃)

### EMBODIMENTS OF INVENTION

The novel aspartyl dipeptide ester derivatives of the invention include the compounds represented by formula (1) and salts thereof.

Amino acids constituting the derivatives are preferably L-isomers in that these are present in nature.

With respect to the compounds of the invention, the following embodiments are preferred.

Compounds of formula (1) wherein R₂ is a hydroxyl group, R₁, R₄, R₅ and R₆ are each a hydrogen atom, and R₇ is a methyl group.

Compounds of formula (1) wherein R₂ is a methoxy group, R₁, R₄, R₅ and R₆ are each a hydrogen atom, and R₇ is a methyl group.

Compounds of formula (1) wherein R₁, R₂, R₄, R₅ and R₆ are each a hydrogen atom, and R₇ is a methyl group.

Compounds of formula (1) wherein R₂ and R₆ are each a hydroxyl group, R₁, R₄ and R₅ are each a hydrogen atom, and R₇ is a methyl group.

Compounds of formula (1) wherein R₁ is a hydroxyl group, R₂ R₄, R₅ and R**₆** are each a hydrogen atom, and R₇ is a methyl group.

Compounds of formula (1) wherein R₁ is a methoxy group, R₂, R₄, R₅ and R₆ are each a hydrogen atom, and R₇ is a methyl group.

Compounds of formula (1) wherein R₁ is an ethoxy group, R₂, R₄ R₅ and R₆ are each a hydrogen atom, and R₇ is a methyl group.

Compounds of formula (1) wherein R₂ and R₇ are each a methyl group, and R₁, R₄, R₅ and R₆ are each a hydrogen atom.

Examples of the salts of the compounds in the invention include salts with alkali metals such as sodium and potassium; salts with alkaline earth metals such as calcium and magnesium; ammonium salts with ammonia; salts with amino acids such as lysine and arginine; salts with inorganic acids such as hydrochloric acid and sulfuric acid; and salts with organic acids such as citric acid and acetic acid. These are included in the derivatives of the invention as described above.

The aspartyl dipeptide ester derivatives of the invention can easily be formed by reductively alkylating aspartame derivatives with cinnamaldehydes having various substituents and a reducing agent (for example, hydrogen/palladium carbon catalyst). Alternatively, the derivatives can be formed by subjecting aspartame derivatives (for example, β-O-benzyl-α-L-aspartyl-L-phenylalaninemethyl ester) having a protective group in a carboxylic acid in the β-position which derivatives can be obtained by the usual peptide synthesis method (Izumiya et al., Basis of Peptide Synthesis and Experiments Thereof, Maruzen, published January 20, 1985) to reductive alkylation with cinnamaldehydes having various substituents and a reducing agent (for example, NaB(OAc)₃H) (A. F. Abdel-Magid et al., Tetrahedron Letters, 31, 5595 (1990)), and then removing the protective group. However, the method of forming the compounds of the invention is not limited thereto. 3-Phenylpropionaldehydes having various substituents or acetal derivatives thereof can of course be used as precursor aldehydes in the reductive alkylation instead of cinnamaldehydes having various substituents.

As a result of a sensory evaluation, the compounds and the salts thereof in the invention were found to have a strong sweetening potency and have taste properties similar to that of sugar. For example, the sweetening potency of N- [N- [3- (3-hydroxy-4-methoxyphenyl)propyl] -L-α-aspartyl]-L-phenylalanine 1-methyl ester was approximately 20,000 times (relative to sugar), that of N-[N-[3-(2-hydroxy-4-methoxyphenyl) propyl] -L-α-aspartyl] -L-phenylalanine 1-methyl ester was approximately 20, 000 times (relative to sugar), that of N-[N-[3-(4-methoxyphenyl)propyl]-L-α-aspartyl]-L-phenylalanine 1-methyl ester was approximately 6,500 times (relative to sugar), and that of N-[N-[3-(3-hydroxy-4-methoxyphenyl)propyl]-L-α-aspartyl]-L-tyrosine 1-methyl ester was approximately 16,000 times (relative to sugar).

With respect to the aspartyl dipeptide derivatives (represented by formula (2)) formed, the structures and the results of the sensory evaluation are shown in Table 1.

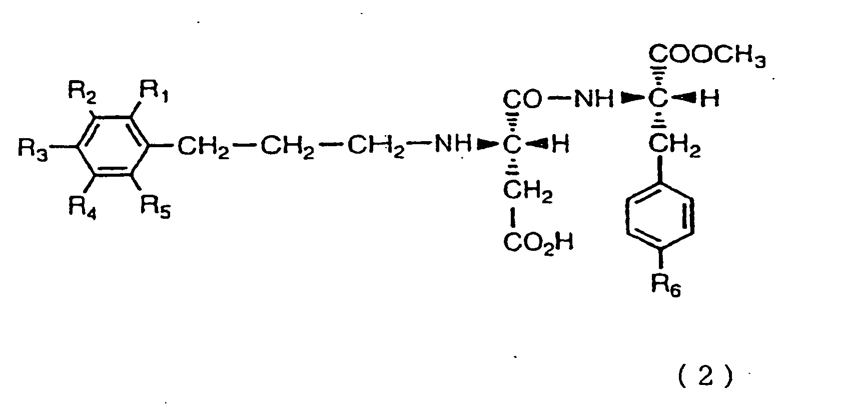

**Table 1**

| Structures and sweetening potency of aspartyl dipeptide ester derivatives | | | | | | | |
|---|---|---|---|---|---|---|---|
| Compound No. | R₁ | R₂ | R₃ | R₄ | R₅ | R₆ | sweetening potency*⁾ |
| 1 | H | OH | OCH₃ | H | H | H | 20000 |
| 2 | H | OCH₃ | OCH₃ | H | H | H | 2500 |
| 3 | H | H | OCH₃ | H | H | OH | 6500 |
| 4 | H | OH | OCH₃ | H | H | H | 16000 |
| 5 | OH | H | OCH₃ | H | H | H | 20000 |
| 6 | OCH₃ | H | OCH₃ | H | H | H | 4000 |
| 7 | OCH₂CH₃ | H | OCH₃ | H | H | H | 2500 |
| 8 | H | CH₃ | OCH₃ | H | H | H | 8000 |

| | | | | | | | |
|---|---|---|---|---|---|---|---|
| *) Relative to sweetening potency of a 4% sucrose aqueous solution | | | | | | | |

As understood from the results of Table 1, the novel derivatives in the present invention are excellent in sweetening potency.

When the compounds (including those in the form of a salt) of the invention are used as a sweetener, these may of course be used in combination with other sweeteners unless inviting any special troubles.

When the derivatives of the invention are used as a sweetener, an appropriate carrier and/or an appropriate bulking agent may be used as required. For example, a carrier which has been so far used is available.

The derivatives of the invention can be used as a sweetener or an ingredient therefor, and further as a sweetener for products such as foods and the like to which a sweetness has to be imparted, for example, confectionary, chewing gum, hygiene products, toiletries, cosmetics, pharmaceutical products and veterinary products for animals. Still further, they can be used in a method of imparting a sweetness to the products. This method can be, for example, a conventional method for using a sweetening ingredient for a sweetener in the sweeteners or the method of imparting a sweetness.

### PREFERRED EMBODIMENTS OF INVENTION

The invention is illustrated specifically by referring to the following Examples.

### EXAMPLE 1

### Synthesis of N- [N- [3- (3-hydroxy-4-methoxyphenyl)propyl] -L-α-aspartyl] -L-phenylalanine 1-methyl ester

Five milliliters of a solution of 4N-HCl and dioxane were added to 485 mg (1.0 mmol) of N-t-butoxycarbonyl-β-O-benzyl-α-L-aspartyl-L-phenylalanine methyl ester, and the mixture was stirred at room temperature for 1 hour. The reaction solution was concentrated under reduced pressure. Thirty milliliters of a 5% sodium hydrogencarbonate aqueous solution were added to the residue, and the mixture was extracted twice with 30 ml of ethyl acetate. The organic layer was washed with a saturated aqueous solution of sodium chloride, and dried over anhydrous magnesium sulfate. Then, magnesium sulfate was removed by filtration, and the filtrate was concentrated under reduced pressure to obtain 385 mg of β-O-benzyl-α-L-aspartyl-L-phenylalanine methyl ester as a viscous oil.

The β-O-benzyl-α-L-aspartyl-L-phenylalanine methyl ester (385 mg, 1.0 mmol) was dissolved in 15 ml of THF, and the solution was maintained at 0°C. To this were added 268 mg (1.0 mmol) of 3-benzyloxy-4-methoxycinnamaldehyde, 0.060 ml (1.0 mmol) of acetic acid and 318 mg (1.5 mmol) of NaB(OAc)₃H. The mixture was stirred at 0°C for 1 hour and further overnight at room temperature. To the reaction solution were added 50 ml of a saturated aqueous solution of sodium hydrogen carbonate, and the mixture was extracted twice with 30 ml of ethyl acetate. The organic layer was washed with a saturated aqueous solution of sodium chloride, and dried over anhydrous magnesium sulfate. Then, magnesium sulfate was removed by filtration, and the filtrate was concentrated under reduced pressure. The residue was purified with PTLC (Preparative Thin Layer Chromatography) to obtain 523 mg (0.82 mmol) of N-[M-[3-(3-benzyloxy-4-methoxyphenyl)propenyl]-β-O-benzyl-L-α-aspartyl]-L-phenylalanine 1-methyl ester as a viscous oil.

The N-[N-[3-(3-benzyloxy-4-methoxyphenyl)propenyl]-β-O-benzyl-L-α-aspartyl] -L-phenylalanine 1-methyl ester (523 mg, 0.82 mmol) was dissolved in a mixed solvent of 30 ml of methanol and 1 ml of water, and 200 mg of 10% palladium carbon (water content 50%) were added thereto. The mixture was reduced under a hydrogen stmosphere at room temperature for 3 hours. The catalyst was removed by filtration, and the filtrate was concentrated under reduced pressure. In order to remove an odor adsorbed, the residue was purified with PTLC to obtain 228 mg (0.48 mmol) of N-[N-[3-(3-hydroxy-4-methoxyphenyl)propyl]-L-α-aspartyl]-L-phenylalanine 1-methyl ester as a solid.

¹HNMR(DMSO-d ₆) δ : 1.50 - 1.6 0 (m, 2 H), 2. 15 - 2.4 0 (m, 6H), 2. 87- 2.9 7 (dd. 1H), 3. 0 5-3. 13 (d d, 1H), 3**.**3 7**-**3**.**4 3 (m, 1H), 3. 6 2 ( s , 3H), 3. 71 (s, 3H), 4. 50-4. 60 (m, 1H), 6. 52 (d, 1H). 6.60 (s,1H), 6.79 (d, 1H), 7.18-7.30 (m, 5H), 8. 5 2 (d, 1H), 8. 8 0 (brs, 1H).

E S I -MS 459. 2 (MH⁺)

Sweetening potency (relative to sugar): 20,000 times

### EXAMPLE 2

### Synthesis of N-[N-[3-(3,4-dimethoxyphenyl)propyl]-L-α-aspartyl]-L-phenylalanine 1-methyl ester

Example 1 was repeated except that 3,4-dimethoxycinnamaldehyde was used instead of 3-benzyloxy-4-methoxycinnamaldehyde to obtain N-[N-[3-(3,4-dimethoxyphenyl)propyl]-L-α-aspartyl]-L-phenylalanine 1-methyl ester in a total yield of 48.7% as a solid.

¹HNMR (DMSO-d₆) δ : 1 . 5 2- 1. 6 2 (m, 2H), 2. 1 8 - 2 . 5 0 (m, 6 H), 2. 8 6 - 2 . 7 6 (dd , 1 H ), 3.0 4 - 3 . 1 2 ( d d , 1 H ), 3.37-3.44 (m, 1 H), 3. 6 2 ( s , 3H), 3. 7 1 (s , 3H), 3.73 (s, 3H), 4. 5 2-4. 62 (m, 1H), 6. 6 6 (d, 1H), 6. 7 6 (s, 1H), 6. 8 3 (d, 1H), 7. 1 8- 7. 30 (m, 5H), 8. 50 (d, 1H).

ESI-MS 473.2 (MH⁺)

Sweetening potency (relative to sugar): 2,500 times

### EXAMPLE 3

### Synthesis of N- [N- [3- (4-methoxyphenyl)propyl] -L-α-aspartyl]-L-phenylalanine 1-methyl ester (1)

Example 1 was repeated except that 4-methoxycinnamaldehyde was used instead of 3-benzyloxy-4-methoxycinnamaldehyde to obtain N-[N-[3-(4-methoxyphenyl)propyl]-L-α-aspartyl]-L-phenylalanine 1-methyl ester in a total yield of 50.0% as a solid.

¹HNMR (DMSO-d₆) δ : 1. 5 0- 1. 6 2 (m, 2 H), 2 . 16-2. 48 (m, 6 H), 2. 8 4 - 2 . 94 ( d d , 1 H ), 3. 0 4-3. 12 (dd, 1H), 3. 3 8 - 3. 4 4 (m, 1H), 3. 6 2 (s , 3 H), 3. 7 1 ( s , 3 H), 4 . 5 2 - 4 . 6 2 (m, 1 H), 6 . 8 3 ( d , 2H), 7. 0 8 (d, 2H), 7.17-7.29 (m, 5H), 8. 5 0 (d, 1 H).

ESI-MS 443.3 (MH⁺)

Sweetening potency (relative to sugar): 6,500 times

### EXAMPLE 4

### Synthesis of N-[N-[3-(4-methoxyphenyl)propyl]-L-α-aspartyl]-L-phenylalanine 1-methyl ester (2)

4-Methoxycinnamaldehyde (405 mg, 2.5 mmol), 735 mg (2.5 mmols) of aspartame and 350 mg of 10% palladium carbon (water content 50%) were added to a mixed solvent of 15 ml of methanol and 5 ml of water, and the mixture was stirred under a hydrogen atmosphere overnight at room temperature. The catalyst was removed by filtration, and the filtrate was concentrated under reduced pressure. To the residue were added 30 ml of ethyl acetate, and the mixture was stirred for a while. Then, the insoluble material was collected by filtration. The insoluble material collected was washed with a small amount of ethyl acetate. To this were added 50 ml of a mixed solvent of ethyl acetate and methanol (5:2), and the mixture was stirred for a while. The insoluble material was removed by filtration, and the filtrate was concentrated. Then, the overall residue was solidified. This was dried under reduced pressure, and then recrystallized from a mixed solvent of methanol and water to obtain N-[N-[3-(4-methoxyphenyl)propyl]-L-α-aspartyl]-L-phenylalanine 1-methyl ester in a total yield of 43.4% as a solid.

### EXAMPLE 5

### Synthesis of N-[N-[3-(3-hydroxy-4-methoxyphenyl)propyl]-L-α-aspartyl]-L-tyrosine 1-methyl ester

Example 1 was repeated except that N-t-butoxycarbonyl-β-O-benzyl-α-L-aspartyl-L-tyrosine methyl ester was used instead of N-t-butoxycarbonyl-β-O-benzyl-α-L-aspartyl-L-phenylalanine methyl ester to obtain N-[N-[3-(3-hydroxy-4-methoxyphenyl)propyl]-L-α-aspartyl]-L-tyrosine 1-methyl ester in a total yield of 45.4% as a solid.

¹HNMR(DMSO-d₆) δ : 1. 5 2- 1. 64 (m. 2H), 2. 24-2.4 8 (m, 6 H), 2. 74-2. 84 (dd, 1H), 2. 9 1 - 2 . 9 9 (dd, 1H), 3.47-3.54 (m, 1H), 3. 61 ( s , 3H), 3. 7 2 (s, 3H), 4. 4 5-4. 5 3 (m, 1 H). 6. 5 4 (d, 1H), 6. 6 0 (s, 1 H), 6. 6 5 (d, 2H). 6. 7 9 (d, 1H), 6. 9 8 (d, 2H), 8. 5 4 (d, 1 H), 8. 7 8 (b r s, 1 H), 9. 2 5 ( b r s , 1 H ).

ESI-MS 475.2(MH⁺)

Sweetening potency (relative to sugar): 16,000 times

### EXAMPLE 6

### Synthesis of N-[N- [3-(2-hydroxy-4-methoxyphenyl)propyl] -L-α-aspartyl]-L-phenylalanine 1-methyl ester

Example 1 was repeated except that 2-benzyloxy-4-methoxycinnamaldehyde was used instead of 3-benzyloxy-4-methoxycinnamaldehyde to obtain N-[N-[3-(2-hydroxy-4-methoxyphenyl)propyl]-L-α-aspartyl]-L-phenylalanine 1-methyl ester in a total yield of 54.4% as a solid.

¹HNMR (DMSO-d₆) δ : 1. 52-1. 57 (m, 2 H), 2 . 2 0 - 2 . 3 1 (m, 2 H), 2 . 2 6 - 2 . 4 1 (m, 4 H), 2 . 8 8 - 3.11 (m, 2 H), 3.41-3.44 (m, 1H), 3. 6 2 ( s , 3 H), 3.6 5 ( s , 3H), 4.53-4.59 (m, 1H), 6.28-6.36 (m, 2H), 6. 88-6 . 9 0 (d, 1H), 7. 19-7 . 2 9 (m, 5H), 8. 5 5 (d,1H).

E S I -M S 4 5 9. 3 (MH⁺)

Sweetening potency (relative to sugar): 20,000 times

### EXAMPLE 7

### Synthesis of N- [N- [3- (2,4-dimethoxyphenyl)propyl] -L-α-aspartyl]-L-phenylalanine 1-methyl ester

Example 1 was repeated except that 2,4-dimethoxycinnamaldehyde was used instead of 3-benzyloxy-4-methoxycinnamaldehyde to obtain N-[N-[3-(2,4-dimethoxyphenyl) propyl] -L-α-aspartyl] -L-phenylalanine 1-methyl ester in a total yield of 32.4% as a solid.

¹HNMR (DMSO- d₆) δ : 1.50-1. 5 4 (m. 2 H), 2. 2 0 - 2 . 3 1 (m, 2 H), 2. 2 5 - 2 . 4 3 (m, 4 H), 2. 8 8 -3. 1 2 (m, 2 H), 3. 4 4-3. 8 2 (m, 1H). 3.6 2 (s , 3 H), 3. 7 2 ( s , 3 H), 3. 7 5 ( s , 3H), 4.5 4 - 4. 5 9 (m, 1H). 6 . 4 0 - 6. 5 0 (m, 2 H), 6. 9 6 - 6 . 9 8 (m, 1 H), 7.1 9 - 7 . 2 9 (m, 5 H), 8 . 5 1 (d. 1 H).

E S I -M S 4 7 3. 3 (MH⁺)

Sweetening potency (relative to sugar): 4,000 times

### EXAMPLE 8

### Synthesis of N-(N-[3-(2-ethoxy-4-methoxyphenyl)propyl]-L-α -aspartyl]-L-phenylalanine 1-methyl ester

Example 1 was repeated except that 2-ethoxy-4-methoxycinnamaldehyde was used instead of 3-benzyloxy-4-methoxycinnamaldehyde to obtain N-[N-[3-(2-ethoxy-4-methoxyphenyl)propyl]-L-α-aspartyl]-L-phenylalanine 1-methyl ester in a total yield of 35.6% as a solid.

¹HNMR (DMSO-d₆) δ : 1. 30-1. 34 (t, 3H), 1. 5 0 - 1 . 5 7 (m, 2 H), 2. 1 9-2. 4 1 (m, 2 H), 2.2 4 - 2 . 4 3 (m, 4 H), 2. 8 7 -3. 1 (m, 2H), 3. 3 8 - 3 . 4 2 (m, 1H), 3. 6 2 ( s , 3H). 3. 7 1 ( s , 3 H), 3. 7 0 - 4. 0 3 (q, 2 H), 4. 5 3 - 4. 6 0 (m, 1H), 6. 4.0 - 6. 48 (m. 2 H), 6. 9 6- 6. 98 (m, 1H), 7. 1 9-7. 29 (m, 5 H), 8. 5 1 (d., 1H).

ES I-MS 487.4(MH⁺)

Sweetening potency (relative to sugar): 2,500 times

### EXAMPLE 9

### Synthesis of N- [N- [3-(3-methyl-4-methoxyphenyl)propyl]-L-α -aspartyl]-L-phenylalanine 1-methyl ester

Example 1 was repeated except that 3-methyl-4-methoxycinnamaldehyde was used instead of 3-benzyloxy-4-methoxycinnamaldehyde to obtain N-[N-[3-(3-methyl-4-methoxyphenyl)propyl]-L-α-aspartyl]-L-phenylalanine 1-methyl ester in a total yield of 34.0% as a solid.

¹HNMR (DMSO-d₆) δ : 1. 52-1. 59 (m, 2H), 2. 11 ( s , 3 H), 2. 2 0 - 2 . 3 8 (m, 2 H), 2 . 2 6 - 2 . 4 3 (m, 4H), 2. 8 9- 3. 1 0 (m, 2 H), 3. 3 9- 3. 4 3 (m, 1H), 3. 6 2 (s, 3 H), 3.73 (s, 3H), 4. 5 2-4. 5 9 (m, 1H), 6. 7 9 6. 8 2 (m, 1H), 6. 9 2 -6 . 94 (m, 2H). 7. 19 - 7. 2 8 (m. 5 H), 8. 53 (d, 1H).

E S I - M S 45 7. 4 (MH⁺)

Sweetening potency (relative to sugar): 8,000 times

### EFFECTS OF INVENTION

The novel aspartyl dipeptide ester derivatives of the invention have especially an excellent sweetening potency in comparison with conventional sweeteners. The invention can provide novel chemical substances having excellent taste properties as a sweetener. Accordingly, such novel derivatives in the present invention can be used as a sweetener, and also can impart a sweetness to products such as beverages and foods requiring a sweetness.

## Claims

1. An aspartyl dipeptide ester derivative (including salts thereof) represented by formula (1) 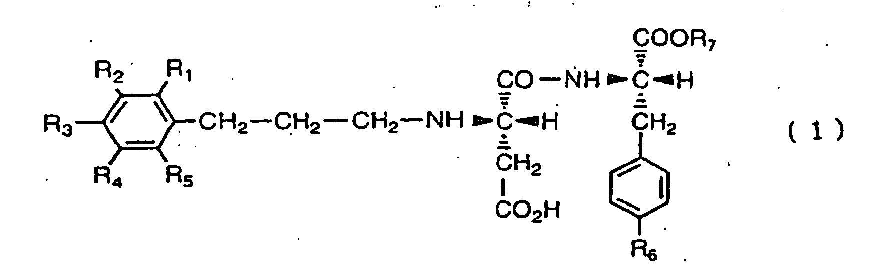 wherein
R₁, R₂, R₄ and R₅, independently from each other, represent a substituent selected from a hydrogen atom, a hydroxyl group, an alkoxy group having from 1 to 3 carbon atoms, an alkyl group having from 1 to 3 carbon atoms and a hydroxyalkyloxy group having 2 or 3 carbon atoms, or R₁ and R₂ together form a methylenedioxy group and R₄ and R₅ independently from each other, each represents any substituent as mentioned above designated for the R₄ and R₅ radicals respectively; R₆ represents a hydrogen atom or a hydroxyl group;
R₃ is methoxy; and
R₇ represents a substituent selected from a methyl group, an ethyl group, an isopropyl group, an n-propyl group and a t-butyl group.

2. A derivative of claim 1, wherein R₂ is a hydroxyl group, R₁ R₄, R₅ and R₆ are hydrogen atoms, and R₇ is a methyl group.

3. A derivative of claim 1, wherein R₂ is a methoxy group, R₁, R₄, R₅ and R₆ are hydrogen atoms, and R₇ is a methyl group.

4. A derivative of claim 1, wherein R₁, R₂, R₄ , R₅ and R₆ are hydrogen atoms, and R₇ is a methyl group.

5. A derivative of claim 1, wherein R₂ and R₆ are hydroxyl groups, R₁, R₄ and R₅ are hydrogen atoms, and R₇ is a methyl group.

6. A derivative of claim 1, wherein R₁ is a hydroxyl group, R₂ , R₄, R₅ and R₆ are hydrogen atoms, and R₇ is a methyl group.

7. A derivative of claim 1, wherein R₁ is a methoxy group, R₂ R₄, R₅ and R₆ are hydrogen atoms, and R₇ is a methyl group.

8. A derivative of claim 1, wherein R₁ is an ethoxy group, R₂, R₄, R₅ and R₆ are hydrogen atoms, and R₇ is a methyl group.

9. A derivative of claim 1, wherein R₂ and R₇ are methyl groups, and R₁, R₄ , R₅ and R₆ are hydrogen atoms.

10. A sweetener or products such as foods having a sweetness, comprising at least one derivative selected from the derivatives of any preceding claim as an active ingredient.

11. A sweetener of product as defined in claim 10, further comprising a carrier and /or a bulking agent for sweeteners.

12. The use of a derivative as claimed in any one of claims 1 to 9 as a sweetener.

## Patentansprüche

1. Aspartyldipeptidesterderivat (einschließlich dessen Salze) der Formel (1) 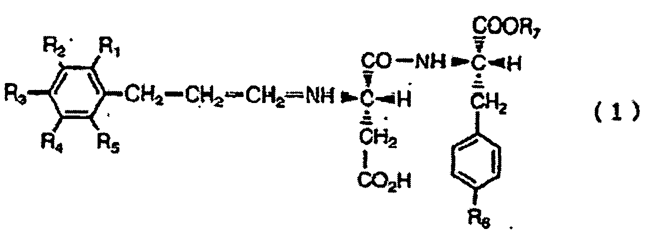 worin
R₁, R₂, R₄ und R₅ unabhängig voneinander einen Substituenten darstellen, der unter einem Wasserstoffatom, einer Hydroxygruppe, einer Alkoxygruppe mit 1 bis 3 Kohlenstoffatomen, einer Alkylgruppe mit 1 bis 3 Kohlenstoffatomen und einer Hydroxyalkyloxygruppe mit 2 oder 3 Kohlenstoffatomen ausgewählt ist, oder R₁ und R₂ zusammen eine Methylendioxygruppe darstellen und R₄ und R₅ unabhängig voneinander jeweils einen beliebigen Substituenten darstellen, der vorstehend für die R₄ bzw. R₅-Radikale genannt wurde;
R₆ ein Wasserstoffatom oder eine Hydroxygruppe darstellt;
R₃ Methoxy ist; und
R₇ einen Substituenten darstellt, der unter einer Methylgruppe, Ethylgruppe, Isopropylgruppe, n-Propylgruppe und t-Butylgruppe ausgewählt ist.

2. Derivat nach Anspruch 1, worin R₂ eine Hydroxygruppe ist, R₁, R₄, R₅ und R₆ Wasserstoffatome sind und R₇ eine Methylgruppe ist.

3. Derivat nach Anspruch 1, worin R₂ eine Methoxygruppe ist, R₁, R₄, R₅ und R₆ Wasserstoffatome sind und R₇ eine Methylgruppe ist.

4. Derivat nach Anspruch 1, worin R₁, R₂, R₄, R₅ und R₆ Wasserstoffatome sind und R₇ eine Methylgruppe ist.

5. Derivat nach Anspruch 1, worin R₂ und R₆ Hydroxygruppen sind, R₁, R₄ und R₅ Wasserstoffatome sind und R₇ eine Methylgruppe ist.

6. Derivat nach Anspruch 1, worin R₁ eine Hydroxygruppe ist, R₂, R₄, R₅ und R₆ Wasserstoffatome sind und R₇ eine Methylgruppe ist.

7. Derivat nach Anspruch 1, worin R₁ eine Methoxygruppe ist, R₂, R₄, R₅ und R₆ Wasserstoffatome sind und R₇ eine Methylgruppe ist.

8. Derivat nach Anspruch 1, worin R₁ eine Ethoxygruppe ist, R₂, R₄, R₅ und R₆ Wasserstoffatome sind und R₇ eine Methylgruppe ist.

9. Derivat nach Anspruch 1, worin R₂ und R₇ Methylgruppen sind und R₁, R₄, R₅ und R₆ Wasserstoffatome.

10. Süßmittel oder Produkte, wie Nahrungsmittel mit Süßkraft, welche mindestens ein Derivat, das unter den Derivaten nach einem der vorstehenden Ansprüche ausgewählt ist, als aktiven Bestandteil enthalten.

11. Süßmittel oder Produkt nach Anspruch 10, das außerdem einen Träger und/oder ein Füllmittel für Süßmittel enthält.

12. Verwendung eines Derivats nach einem der Ansprüche 1 bis 9 als Süßmittel.

## Revendications

1. Dérivé d'aspartyl dipeptide ester (y compris ses sels) représenté par la formule (1) 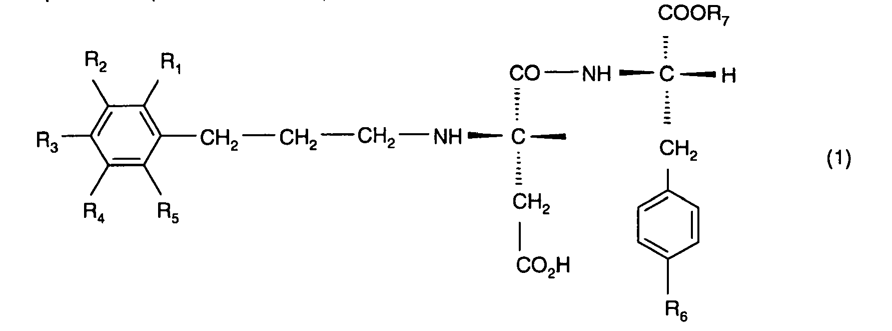 où
R₁, R₂, R₄ et R₅, indépendamment les uns des autres, représentent un substituant choisi parmi un atome d'hydrogène, un groupe hydroxyle, un groupe alcoxy ayant de 1 à 3 atomes de carbone, un groupe alkyle ayant de 1 à 3 atomes de carbone et un groupe hydroxyalkyloxy ayant 2 ou 3 atomes de carbone, ou bien R₁ et R₂ forment ensemble un groupe méthylènedioxy et R₄ et R₅, indépendamment l'un de l'autre, représentent chacun tout substituant tel que mentionné ci-dessus désigné pour les radicaux R₄ et R₅ respectivement ; R₆ représente un atome d'hydrogène ou un groupe hydroxyle ;
R₃ est méthoxy ; et
R₇ représente un substituant choisi parmi un groupe méthyle, un groupe éthyle, un groupe isopropyle, un groupe n-propyle et un groupe t-butyle.

2. Dérivé selon la revendication 1, où R₂ est un groupe hydroxyle, R₁, R₄ et R₅ et R₆ sont des atomes d'hydrogène et R₇ est un groupe méthyle.

3. Dérivé selon la revendication 1, où R₂ est un groupe méthoxy, R₁, R₄, R₅ et R₆ sont des atomes d'hydrogène et R₇ est un groupe méthyle.

4. Dérivé selon la revendication 1, où R₁, R₂, R₄, R₅ et R₆ sont des atomes d'hydrogène et R₇ est un groupe méthyle.

5. Dérivé selon la revendication 1, où R₂ et R₆ sont des groupes hydroxyle, R₁, R₄ et R₅ sont des atomes d'hydrogène et R₇ est un groupe méthyle.

6. Dérivé selon la revendication 1, où R₁ est un groupe hydroxyle, R₂, R₄, R₅ et R₆ sont des atomes d'hydrogène et R₇ est un groupe méthyle.

7. Dérivé selon la revendication 1, où R₁ est un groupe méthoxy, R₂, R₄, R₅ et R₆ sont des atomes d'hydrogène et R₇ est un groupe méthyle.

8. Dérivé selon la revendication 1, où R₁ est un groupe éthoxy, R₂, R₄, R₅ et R₆ sont des atomes d'hydrogène et R₇ est un groupe méthyle.

9. Dérivé selon la revendication 1, où R₂ et R₇ sont des groupes méthyle, et R₁, R₄, R₅ et R₆ sont des atomes d'hydrogène.

10. Edulcorant de produits comme des aliments ayant une douceur, comprenant au moins un dérivé choisi parmi les dérivés selon l'une quelconque des revendications précédentes comme ingrédient actif.

11. Edulcorant de produit selon la revendication 10 comprenant en outre un support et/ou un diluant pour édulcorants.

12. Utilisation d'un dérivé selon l'une quelconque des revendications 1 à 9 comme édulcorant.
